# EUROPEAN PATENT SPECIFICATION

(11) **EP 2 252 858 B1**
(45) Date of publication and mention of the grant of the patent: **25.07.2012**
(21) Application number: 08873223.5
(22) Date of filing: 31.10.2008
(51) Int. Cl.: G01L 5/00, G01N 3/40, G01N 3/58, A01G 23/00

(54) **METHOD AND DEVICE FOR DETERMINING THE VOLUME OF WOOD**
VERFAHREN UND EINRICHTUNG ZUR BESTIMMUNG VON DEM VOLUMEN VON HOLZ
PROCÉDÉ ET DISPOSITIF DE DÉTERMINATION DU VOLUME DE BOIS

(30) Priority: 12.03.2008 FI 20080201
(43) Date of publication of application: 24.11.2010
(73) Proprietor: Usenius, Jussi-Pekka, 40950 Muurame (FI); Usenius, Hannu, 01900 Nurmijärvi (FI)
(72) Inventor: Usenius, Jussi-Pekka, 40950 Muurame (FI); Usenius, Hannu, 01900 Nurmijärvi (FI)
(74) Representative: Pitkänen, Hannu Alpo Antero
(86) International application number: PCT/FI2008/050622
(87) International publication number: WO 2009/112627

(56) References cited:
- WO-A1-01/84910
- WO-A1-87/07203
- WO-A1-2008/025881
- DE-C1- 4 343 520
- JP-A- 6 331 532
- SU-A1- 640 149
- SU-A1- 917 013
- SU-A1- 1 276 928
- SU-A1- 1 276 928
- SU-A1- 1 509 644
- SU-A1- 1 509 644
- SU-A1- 1 597 620
- SU-A1- 1 597 620
- US-A- 4 249 414
- US-A- 5 043 907

## Description

The invention relates to a method for determining of volume of wood which is specified in the preamble of claim 1. Furthermore, the invention relates to a device for the determining of volume of wood which is specified in the preamble of the first device claim.

Mechanic harvesting of fuel wood has become more and more common in recent years. Harvested standing timber is principally wood of small diameter, the base diameter being about 5-25 cm. The harvested wood usually goes for processing through chipping or chopping, whereby a cut surface created in the wood in connection with felling does not have similar quality requirements as a cut surface of raw wood going to the production of sawn timber. Because of this, at the felling heads of a harvester developed for harvesting fuel wood there is most usually a cutting knife or equivalent operating in the so-called guillotine style, cutting with a pressing motion. Then, the volume of harvested wood is not measured with similar measuring devices determining wood volume as when harvesting delimbed log or pulp-wood, but usually the volume of harvested wood is estimated either from a stack on the roadside by measuring the perimeter or by defining the weight of wood on a weigher fastened to the crane of a forwarder or a timber carrier, sometimes also a harvester. The specific weight of wood is strongly dependent on its water content, due to which considerable inaccuracies are related to determining the volume of wood from its weight. In harvesting fuel wood, moisture content or net energy content has also been used in addition to volume as the basis of measuring and pricing wood, but this is awkward.

US 5043907 describes a band saw having a controller responsive to sensing signals as to operate parameters of the saw to generate a material distribution profile during a first cur o piece of bar stock. This band saw is for cutting of a metallic bar stock. WO 01/84910 describe a method and arrangement for adjusting feed rate of a cross-cur saw. SU-1597620, SU-1509644, SU 1276928 and WO 2008/025881 describe also devices for cutting of woods.

The object of the invention is to introduce a method and a device for determining the volume of wood by means of which above disadvantages are eliminated. Particularly, the object of the invention is to introduce a method and a device by means of which the diameter, cut surface area and volume of a tree can be reliably determined when cutting the tree.

In the volume measurement principle described here, the harvester measures the diameter or area of the cut surface of a tree being cut directly from the operation of the cutting knife, of which data it is also possible to deduce the total volume of cut wood. The measurement takes place during cutting and the data is immediately available. Particularly, the method and the device according to the invention are applicable to measuring and determining the wood diameter, cut surface area and wood volume. Furthermore, the method and the device are applicable to the simultaneous measurement and determination of the combined diameter, cut surface area and volume of two or more tree trunks.

The invention will now be described in more detail with reference to the accompanying drawings in which
Fig. 1 schematically shows the structure and method of a measuring device,
Fig. 2 shows thrust required by a cutting means as a function of time,
Fig. 3 shows the connection of cutting pressure and diameter with unseasoned spruce, and
Fig. 4 shows the connection of cutting pressure and diameter with unseasoned birch.

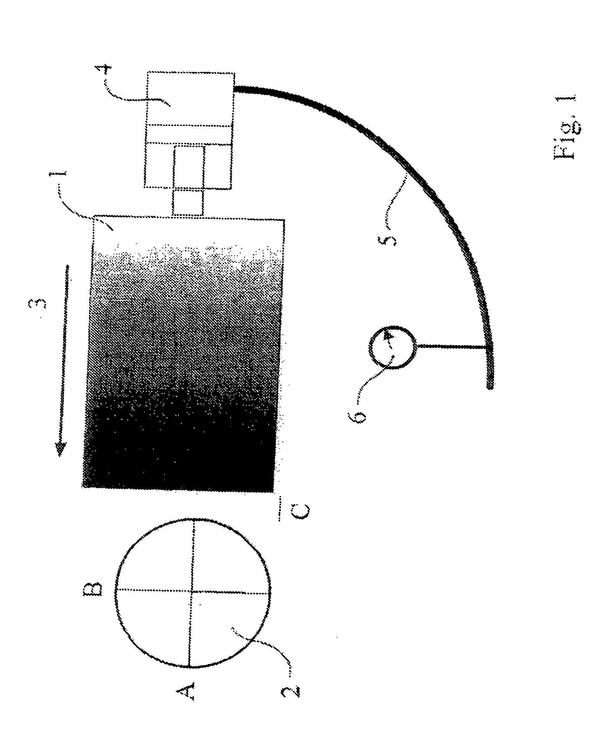

A method according to the invention and the operating principle and structure of a measuring device according to the invention are schematically shown in Fig. 1. The cutting device includes a cutting means 1 and a power unit 4 for moving the cutting means. Furthermore, the measuring device includes a monitoring and calculation apparatus connected to the parts of the device, which is not shown in the figures. As the cutting means, there is a means appropriate for the purpose provided with a cutting blade, such as e.g. a cutting knife, plate or some other known means. As the power unit, there is a device appropriate for the purpose which is arranged to move the cutting means against a tree 2 with required force for cutting the tree. As such a device, a hydraulic cylinder is usually used, but also other power units are possible. The power unit, such as the hydraulic cylinder, is arranged to press the cutting means against the tree, whereby the tree is cut in the so-called guillotine style. The travel of the cutting means can be linear (arrow 3) or rotatory in relation to the tree. The pressing force of the hydraulic cylinder is directly proportional to the pressure of hydrostatic fluid which in Fig. 1 is measured by a pressure gauge 6 set in a pipeline 5 bringing fluid in the hydraulic cylinder. In another embodiment, the pressing force can be measured by a power sensor directly connected to the cutting blade.

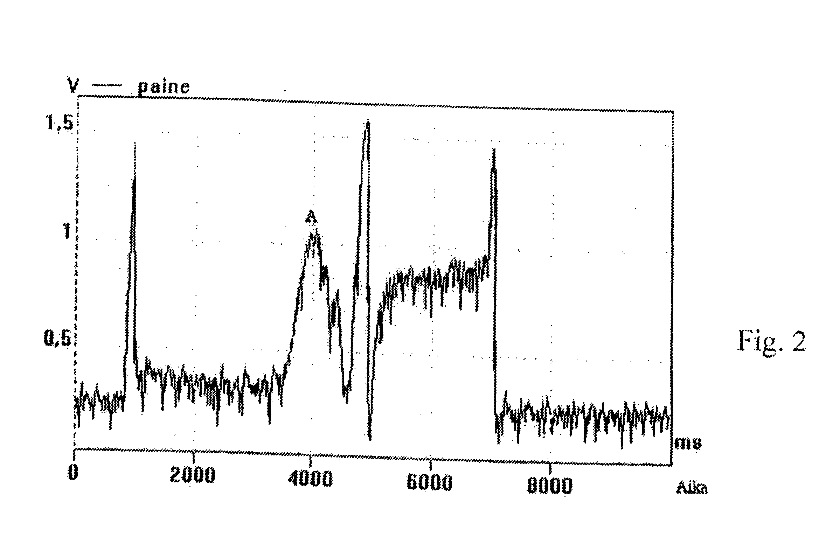

Fig. 2 shows a thrust pressure curve of a travel cylinder of the cutting means. This clearly illustrates that when the cutting means, such as a cutting knife 3, is moved towards the tree, it is first able to move freely, whereby the thrust required for moving is small. When the cutting means meets the surface of the tree, the thrust required for moving the cutting means increases substantially, varies according to the properties of wood and then decreases again to the initial level after the tree has been cut.

As the travel speed of the cutting knife is known for the whole time of cutting, it is possible to estimate a diameter A (in Fig. 1) of the tree in the travel direction of the cutting knife from the duration of the increase of pressing force. This can also be calculated from a dead travel section C (in Fig. 1) of the cutting knife between the blade of the cutting knife and the surface contact of the tree by deducting this from the total travel length of the knife. As the force required for moving the cutting knife is at its greatest when the most part of the blade length of the cutting knife takes part in cutting the tree, it is possible to estimate a diameter B (in Fig. 1) perpendicular against the travel of the cutting knife of the tree from the greatest pressing force of the cutting knife during cutting. In the case of hydraulic transmission, this can be calculated from the highest pressure peak of hydraulic fluid during cutting. From these dimensions, of one separately or using two dimensions together, it is possible to calculate the area of the cut surface of wood when presuming the cross section of wood circular or elliptic. The area of the peak (in Fig. 2) of the force curve, the pressure curve in the case of hydraulic transmission, during cutting reflects the cut surface area of wood as such.

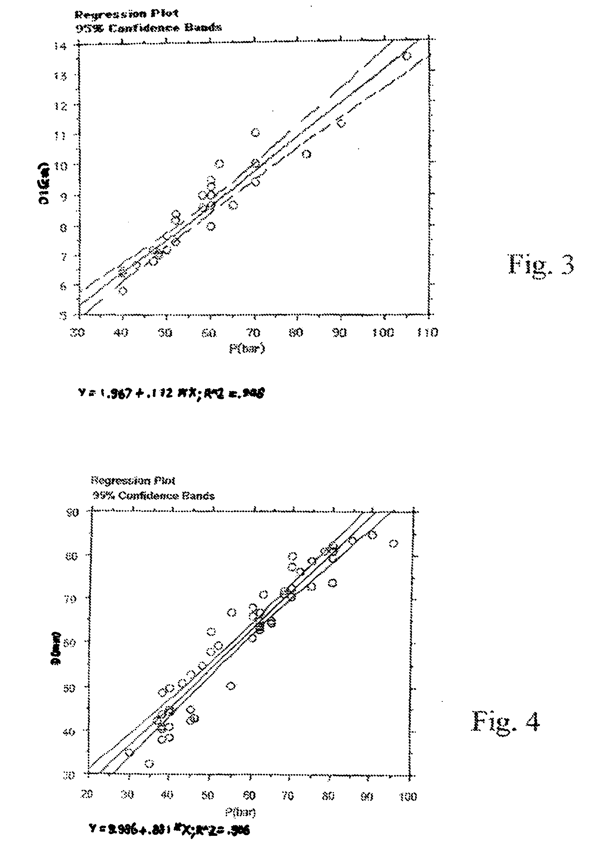

The ratio of the maximal hydraulic pressure to the cut diameter of spruce and birch in the cutting tests of a prototype of the measuring device is shown in Figs. 3 and 4, from which it is seen that in the typical diameter range 4-8 cm the statistical 95-% confidence interval of the diameter of wood is about 5 mm, i.e., about 10% of the diameter.

The wood volume can be calculated from the diameter of the stub using the Laasasenaho equations, among others. The standard error of volume deduced from the stub diameter with the Finnish wood species pine, spruce and birch is respectively 17.2%, 18.7% and 18.8%. If length data of standing timber is added to the measurement, the standard error is respectively 7.2%, 7.6% and 8.5%. If the cut diameter obtained from the height of 6 m is added to the calculation, the standard error is respectively 3.5%, 3.4% and 3.5%. If the tree trunk is cut as a cut-to-size log at two cutting points, the volume of the log produced can be calculated by applying the model of a tapered cylinder. When measuring a larger wood volume, e.g. when calculating daily output, it is possible to add all cross-section measurements based on cutting and, when the combined total length of the wood grade is observed, the average total wood volume can be worked out. Then, a measuring error related to the measurement of one log is avoided and the measuring accuracy of a large wood volume is improved.

Comparing Figs. 3 and 4, it is found that with spruce the cutting pressure equates about 30% thicker wood than with birch. This is because the compression strength perpendicular against the birch grains is about double compared to Finnish soft wood species. From this follows that the measuring device is able to distinguish between birch and the softer wood species by comparing the maximal cutting force of the cutting knife with the travel-directional diameter data defined from the free travel when the tree is assumed circular of its trunk. This can be utilised when wishing to register the proportion of birch from the other wood. The use value of birch is somewhat different as fuel wood compared to other common wood species.

When utilising the method and apparatus according to the invention, it is possible to take two or more (thin) trunks into the cutting device, whereby when cutting them the pressing force of the power unit is measured in a way described above during the travel of the cutting means and the cutting of the trees and from the pressing force the property values of trees are calculated. This way, it is possible to quicken the harvesting of wood and simultaneously also to be able to obtain data reliable enough and to monitor the felling work. Equivalently, if a single tree branches before topping, in which there is thus a multi-trunk cutting event, the method gives also in this case data on the combined wood area of the topping point and the determination can be used in the calculation of the total wood volume.

The measurement principle of the device includes confusing factors which are mainly caused by the biological-physical properties of wood. The elasticity of wood varies somewhat according to moisture and temperature, but this effect can be minimised in the measuring device by compiling reference values which observe the seasons and the outside temperature. A branch, rot or other flaw of wood occurring at the cutting point can give an incorrect measuring result which can interfere with the measuring result of a single merchantable log, but the effect of these local flaws on the measuring result of a total wood batch decreases due to averaging as the number of measuring events increase.

With this method, the practical measuring accuracy in defining the volume of merchantable wood is about 10-30%. Even though the variation is this large, the measuring result can be used e.g. in estimating a daily cut output.

The invention is not limited to the described advantageous embodiments, but it can vary within the scope of the inventive idea presented in the claims.

## Claims

1. A method for determining the volume of wood, in which a cutting means (1) is moved by means of a power unit (4) against a tree (2) and the tree is cut by pressing the cutting means against the tree with required force, ***characterised* in that** the pressing force of the power unit is measured during the travel of the cutting means and the cutting of the tree and from the pressing force are calculated the area of the cut surface of wood and the volume of cut wood.

2. A method according to claim 1, in which the cutting means (1) is moved by means of a hydraulic power unit (4), ***characterised* in that** the pressure of hydrostatic fluid is measured by a pressure gauge (6) set in a pipeline (5) bringing fluid in the power unit and, when the pressing force of the hydraulic cylinder is directly proportional to the pressure of the hydrostatic fluid, from the pressing force are calculated the area of the cut surface of wood and the volume of cut wood.

3. A method according to claim 1, ***characterised* in that** the pressing force is measured by a power sensor directly connected to the cutting blade.

4. A measuring device for determining the volume of wood during the cutting of a tree, whereby the cutting device includes a cutting means (1) and a power unit (4) for moving the cutting means for cutting the tree, ***characterised* in that** the measuring device contains a gauge (6) or a sensor for measuring the pressing force of the power unit during the travel of the cutting means and a means for measuring the area of the cut wood by means of the pressing force and the travel of the cutting means and the means for deteremining the volume of cut wood.

## Patentansprüche

1. Verfahren zum Bestimmen des Volumens von Holz, wobei ein Schneidmittel (1) mithilfe einer Antriebseinheit (4) gegen einen Baum (2) bewegt wird und der Baum durch Pressen des Schneidmittels mit erforderlicher Kraft gegen den Baum geschnitten wird, **dadurch gekennzeichnet, dass** die Presskraft der Antriebseinheit während des Wegs des Schneidmittels und des Schneidens des Baums gemessen wird und aus der Presskraft die Oberfläche der Holzschnittfläche und das Volumen geschnittenen Holzes berechnet wird.

2. Verfahren nach Anspruch 1, wobei das Schneidmittel (1) durch eine hydraulische Antriebseinheit (4) bewegt wird, **dadurch gekennzeichnet, dass** der Druck von hydrostatischem Fluid durch einen Druckmesser (6) gemessen wird, der in einer Rohrleitung (5) eingerichtet ist, die Fluid in die Antriebseinheit einbringt, und, wenn die Presskraft des hydraulischen Zylinders direkt proportional zum Druck des hydrostatischen Fluids ist, aus der Presskraft die Oberfläche der Holzschnittfläche und das Volumen geschnittenen Holzes berechnet wird.

3. Verfahren nach Anspruch 1, **dadurch gekennzeichnet, dass** die Presskraft durch einen Leistungssensor gemessen wird, der direkt mit dem Schneidblatt verbunden ist.

4. Messvorrichtung zum Bestimmen des Volumens von Holz während des Schneidens eines Baums, wobei die Schneidvorrichtung ein Schneidmittel (1) und eine Antriebseinheit (4) zum Bewegen des Schneidmittels zum Schneiden des Baums enthält, **dadurch gekennzeichnet, dass** die Messvorrichtung ein Messgerät (6) oder einen Sensor zum Messen der Presskraft der Antriebseinheit während des Wegs des Schneidmittels und ein Mittel zum Messen der Oberfläche des geschnittenen Holzes mithilfe der Presskraft und des Wegs des Schneidmittels und das Mittel zum Bestimmen des Volumens geschnittenen Holzes enthält.

## Revendications

1. Procédé de détermination du volume du bois, dans lequel un moyen de coupe (1) est déplacé au moyen d'une unité d'alimentation (4) contre un arbre (2), et l'arbre est coupé en appuyant le moyen de coupe contre l'arbre avec la force requise, **caractérisé en ce que** la force de pression de l'unité d'alimentation est mesurée pendant le trajet du moyen de coupe et la coupe de l'arbre, et à partir de la force de pression, sont calculés l'aire de la surface coupée du bois et le volume de bois coupé.

2. Procédé selon la revendication 1, dans lequel le moyen de coupe (1) est déplacé au moyen d'une unité d'alimentation hydraulique (4), **caractérisé en ce que** la pression du fluide hydrostatique est mesurée par un manomètre (6) placé dans une canalisation (5) amenant du fluide à l'unité d'alimentation et, lorsque la force de pression du vérin hydraulique est directement proportionnelle à la pression du fluide hydrostatique, à partir de la force de pression sont calculés l'aire de la surface coupée du bois et le volume de bois coupé.

3. Procédé selon la revendication 1, **caractérisé en ce que** la force de pression est mesurée par un capteur de puissance directement relié à la lame de coupe.

4. Dispositif de mesure pour déterminer le volume de bois pendant la coupe d'un arbre, le dispositif de coupe comprenant un moyen de coupe (1) et une unité d'alimentation (4) pour déplacer le moyen de coupe pour qu'il coupe l'arbre, **caractérisé en ce que** le dispositif de mesure comporte un manomètre (6) ou un capteur pour mesurer la force de pression de l'unité d'alimentation pendant le trajet du moyen de coupe et un moyen pour mesurer l'aire du bois coupé via la force de pression et le trajet du moyen de coupe et le moyen pour déterminer le volume de bois coupé.
